# EUROPÄISCHE PATENTSCHRIFT

(11) **EP 0 928 193 B1**
(45) Veröffentlichungstag und Bekanntmachung des Hinweises auf die Patenterteilung: **12.06.2002**
(21) Anmeldenummer: 97910293.6
(22) Anmeldetag: 16.09.1997
(51) Int. Cl.: A61K 31/44, A61P 17/00

(54) **VERWENDUNG VON 1-HYDROXY-2-PYRIDONEN ZUR BEHANDLUNG VON HAUTINFEKTIONEN**
USE OF 1-HYDROXY-2-PYRIDONES FOR THE TREATMENT OF SKIN DISEASES
UTILISATION DE 1-HYDROXY-2-PYRIDONES POUR LE TRAITEMENT D'INFECTIONS CUTANEES

(30) Priorität: 27.09.1996 DE 19639817
(43) Veröffentlichungstag der Anmeldung: 14.07.1999
(73) Patentinhaber: Aventis Pharma Deutschland GmbH, 65929 Frankfurt am Main (DE)
(72) Erfinder: BOHN, Manfred, D-65719 Hofheim (DE); KRAEMER, Karl, Theodor, D-63225 Langen (DE); MARKUS, Astrid, D-65835 Liederbach (DE)
(86) Internationale Anmeldenummer: EP9705069
(87) Internationale Veröffentlichungsnummer: WO9813043

(56) Entgegenhaltungen:
- EP-A- 0 218 410
- EP-A- 0 646 369
- EP-A- 0 649 660
- EP-A- 0 680 745
- WO-A-96/13247
- DE-A- 3 140 954
- FR-A- 2 685 638
- GB-A- 2 208 149
- HÄNEL: "A comparison of bifonazole and ciclopiroxolamine : in-vitro, animal and clinical studies" MYCOSES, Bd. 31, Nr. 12, 1988, Seiten 632-640, XP002052266

## Beschreibung

Infektionen der Haut werden im überwiegenden Maße durch hautpathogene Bakterien oder Pilze hervorgerufen. Ihre Behandlung erfolgt - in Abhängigkeit vom jeweiligen Erreger - entweder mit antibakteriellen oder mit antimykotischen Mitteln.

Staphylokokken und Streptokokken sind in ca. 70 % aller Fälle Ursache von bakteriellen Infektionen der Haut. Als weiterer wichtiger Erreger von bakteriellen Hautinfektionen sind Proteus sp. zu nennen. Andere aerob und anaerob wachsende Bakterien wie Enterokokken, Escherichia coli, Pseudomonas aeruginosa und Ktebsiellen kommen als Erreger von Hautinfektionen weitaus seltener in Frage.

Hefen dagegen haben als Erreger von Hautinfektionen in jüngerer Zeit deutlich an Bedeutung gewonnen, insbesondere bei Immunsupprimierten, bei denen die mucocutane und systemische Ausbreitung der Hefen ein therapeutisches Problem darstellen können.

Da Bakterien in der Regel keine nennenswerte Keratinaseaktivität, die zum Angehen einer Infektion erforderlich ist, besitzen, sind Pilzinfektionen häufig Ausgangspunkt für das Entstehen von bakteriellen Sekundärinfektionen.

Die vorliegende Erfindung betrifft daher Substanzen, die zur topischen Behandlung sowohl von Pilzinfektionen als auch von bakteriellen Infektionen der Haut geeignet sind. Topische Breitspektrumantiinfektiva gemäß der vorliegenden Erfindung standen bislang als Monopräparate für die Behandlung von Hautinfektionen nicht zur Verfügung.

Bei der Auswahl von Mitteln zur antibakteriellen Therapie müssen u.a. insbesondere Resistenzentwicklungen berücksichtigt werden. Vor allem bei längerer Behandlung ist durch Wundabstriche das Erregerspektrum zu bestimmen und sein Verhalten gegenüber den zur Anwendung kommenden Mitteln zu kontrollieren. Weiterhin muß auf Kontaktsensibilisierungen und Unverträglichkeitsreaktionen geachtet werden. Vor allem bei Neomycin und Gentamycin, die seit vielen Jahren in der Behandlung von Hautinfektionen verwendet werden, ist die Allergisierungsgefahr hoch.

Für die überaus häufigen Staphylokokkeninfekte der Haut werden häufig neben Gentamycin, auch Erythomycin und Clindamycin eingesetzt. Sie kommen sowohl lokal, vorwiegend in der Aknetherapie, als auch systemisch zur Anwendung.

Bedingt durch die seit vielen Jahren ausgeübte systemische Anwendung haben sich jedoch sowol gegenüber Gentamycin als auch gegenüber Erythromycin und Clindamycin im hohen Maße - selbst gegen moderne Gyrasehemmer, wie z.B. Ofloxacin - therapieresistente Bakterienstämme entwickelt. In einer retrospektiven Studie wurde von Th. Forssman et al. (H + G Band 69, Heft 12, 1994, S. 828 - 832) die Antibiotika-Resistenz von Propionibacterium acnes und Staphylococcus epidermidis bei Aknepatienten, die antibiotisch vorbehandelt waren, analysiert.

Die Untersuchungen zeigen, daß bezüglich der Propionibakterien gegen Erythromycin in 36 % und gegen Clindamycin in 11 % der Fälle Resistenzen gefunden wurden. Bei Staphylococcus epidermidis wurden gegen Erythromycin in 90 % und gegen Clindamycin in 40 % der Fälle Resistenzen festgestellt.

Auch gibt die steigende Anzahl von Resistenzen von Enterokokken gegenüber Gentamycin (bis zu 50 % in Isolaten verschiedener Zentren) zu Bedenken Anlaß, zumal die gleichen Stämme auch gegen viele andere Substanzen, einschließlich Vancomycin, resistent sind (Martindale 30. Ausgabe, 1993, S. 171,2).

Das gleiche Problem besteht bei Gentamycin resistenten Staphylococcus aureus Stämmen, die in der Regel auch gegen Methicillin und Ofloxacin unempfindlich sind (Martindale 30. Ausgabe, 1993, S. 171,2 / eigene Untersuchungen).

Es ist ferner aus der Literatur bekannt, daß sich unter den herkömmlichen Antibiotika im zunehmenden Maße Kreuzresistenzen entwickeln. So wurde u.a. bei Patienten, die nur mit Erythromycin vorbehandelt waren, in 20 % der Fälle auch eine Resistenz gegenüber Clindamycin beobachtet.

Aus den geschilderten Gründen heraus gilt es heute nicht mehr als Therapiestandard, systemisch zur Anwendung kommende Antibiotika auch topisch einzusetzen.

Auf der Suche nach einem neuen Therapiestandard für topisch anzuwendende antibiotisch wirksame Substanzen wurde nun überraschend gefunden, daß sich Substanzen aus der Klasse der 1-Hydroxy-2-pyridone, die bislang ausschließlich als Antimykotika Eingang in die Therapie gefunden haben, auch ausgezeichnet zur topischen Behandlung von bakteriellen Hautinfektionen eignen.

in neueren Versuchen konnte nämlich gezeigt werden, daß 1-Hydroxy-2-pyridone gegenüber den bei Hautinfektionen vorkommenden Bakterienarten, insbesondere auch gegen antibiotikaresistente Stämme, ein lückenloses Wirkspektrum aufweisen. In Kombinationen mit den bereits bekannten antimykotischen Eigenschaften der 1-Hydroxy-2-pyridone ist dies für die erfolgreiche Behandlung von Hautinfektionen ein außerordentlich wichtiger Befund, da die bislang obligate Bakterienidentifizierung mit anschließender Resistenzprüfung bei Behandlung mit den erfindungsgemäßen Substanzen nicht mehr erforderlich ist, was letztendlich auch unter anderem zu einer wesentlichen Reduzierung der Behandlungskosten führt.

Die Erfindung betrifft daher die Verwendung von 1-Hydroxy-2-pyridonen der Formel 1, 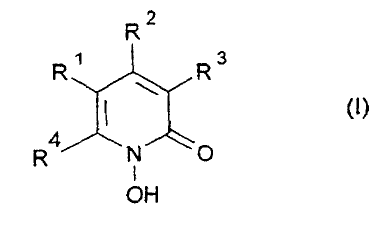 worin R¹, R² und R³, die gleich oder verschieden sind, Wasserstoffatom oder Alkyl mit 1 - 4 Kohlenstoffatomen bedeuten, und
R⁴ einen gesättigten Kohlenwasserstoffrest mit 6 bis 9 Kohlenstoffatomen oder einen Rest der Formel II bedeutet 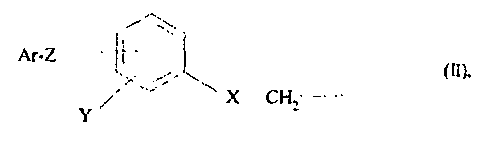 wobei
- X: S oder O bedeutet,
- Y: Wasserstoffatom oder bis zu 2 Halogenatome wie Chlor und/oder Brom bedeutet,
- Z: eine Einfachbindung oder die zweiwertigen Reste O, S, -CR²-(R = H oder (C₁-C₄)-Alkyl) oder andere zweiwertige Reste mit 2- 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder (C₁-C₄)-Alkylgruppen abgesättigt sind, bedeutet,
- Ar: ein aromatisches Ringsystem mit bis zu zwei Ringen, das durch bis zu drei Reste aus der Gruppe Fluor, Chlor, Brom, Methoxy, (C₁-C₄)-Alkyl, Trifluormethyl und Trifluormethoxy substituiert sein kann, bedeutet, in freier oder in Salz-Form,
zur Herstellung eines Arzneimittels zur topischen Behandlung von Hautinfektionen, die durch Pilze und Bakterien verursacht werden.

In den Resten "Z" sind die C-Kettenglieder vorzugsweise CH₂-Gruppen. Wenn die CH₂-Gruppen durch C₁-C₄-Alkylgruppen substituiert sind, sind CH₃ und C₂H₅ bevorzugte Substituenten. Beispielhafte Reste "Z" sind:
-O-, -S-, -CH₂-, -(CH₂)ₘ- (m = 2 - 10), -C(CH₃)₂-, -CH₂O-, -OCH₂-, -CH₂S-, -SCH₂-, -SCH(C₂H₅)-, -CH=CH-CH₂O-, -O-CH₂-CH=CH-CH₂O-, -OCH₂-CH₂O-, -OCH₂-CH₂CH₂O-, -SCH₂CH₂CH₂S-, -SCH₂CH₂CH₂CH₂O-, -SCH₂CH₂OCH₂CH₂O-, -SCH₂CH₂OCH₂CH₂O-CH₂CH₂S- oder -S-CH₂-C(CH₃)₂-CH₂-S-.

Der Rest "S" bedeutet Schwefelatom, der Rest "O" bedeutet Sauerstoffatom. Der Begriff "Ar" bedeutet Phenyl oder kondensierte Systeme wie Naphthyl, Tetrahydronaphthyl und Indenyl, sowie isolierte Systeme wie solche, die sich vom Biphenyl, Diphenylalkanen, Diphenylethern und Diphenylthioethern ableiten.

In der Formel I ist der Kohlenwasserstoff-Rest R⁴ ein Alkyl- oder Cyclohexylrest, der auch über eine Methylen- oder Äthylengruppe an den Pyridonring gebunden sein oder einen Endomethylgruppe enthalten kann. R⁴ kann auch einen aromatischen Rest darstellen, der jedoch vorzugsweise über wenigstens ein aliphatisches C-Atom an den Pyridonrest gebunden ist.

Wichtige Vertreter der durch die Formel I charakterisierten Verbindungsklasse sind:

6-[4-(4-Chlor-phenoxy)-phenoxymethyl]-1-hydroxy-4-methyl-2-pyridon, 6-[4-(2,4-Dichlor-phenoxy)-phenoxymethyl]-1-hydroxy-4-methyl-2-pyridon, 6-(Biphenylyl-4-oxy-methyl)-1-hydroxy-4-methyl-2-pyridon, 6-(4-Benzyl-phenoxymethyl)-1-hydroxy-4-methyl-2-pyridon, 6-[4-(2,4-Dichlorbenzyloxy)-phenoxy-methyl]-1-hydroxy-4-methyl-2-pyridon, 6-[4-(4-Chlor-phenoxy)-phenoxymethyl]-1-hydroxy-3,4-dimethyl-2-pyridon, 6-[4-(2,4-Dichlor-benzyl)-phenoxymethyl]-1-hydroxy-3,4-dimethyl-2-pyridon, 6-[4-(Cinnamyloxy)-phenoxymethyl]-1-hydroxy-4-methyl-2-pyridon, 1-Hydroxy-4-methyl-6-[4-(4-trifluormethyl-phenoxy)-phenoxymethyl]-2-pyridon, 1-Hydroxy-4-methyl-6-cyclohexyl-2-pyridon, 1-Hydroxy-4-methyl-6-(2,4,4-trimethylpentyl)-2-pyridon, 1-Hydroxy-4-methyl-6-n-hexyl-, -6-iso-hexyl-, -6-n-heptyl- oder-6-iso-heptyl-2-pyridon, 1-Hydroxy-4-methyl-6-octyl- oder -6-iso-octyl-2-pyridon, insbesondere 1-Hydroxy-4-methyl-6-cyclohexylmethyl- oder -6-cyclohexylethyl-2-pyridon, wobei der Cyclohexylrest jeweils auch einen Melhyirest tragen kann, 1-Hydroxy-4-melhyl-6-(2-bicyclo[2,2,1]heptyl)-2-pyridon, 1-Hydroxy-3,4-dimethyl-6-benzyl- oder -6-dimethylbenzyl-2-pyridon oder 1-Hydroxy-4-methyl-6-(β-phenyl-ethyl)-2-pyridon.

Der Begriff "gesättigt" bezeichnet hierbei solche Reste, die keine aliphatischen Mehrfachbindungen, also keine ethylenischen oder acetylenischen Bindungen enthalten. Unter dem Begriff "topisch" wird die lokale Wirkung auf der Haut verstanden. Der Begriff "Pilz" bedeutet alle chlorophyllfreie Zellen mit Cellulose oder Chitin in den Zellwänden, die Chromosomen im Zellkern enthalten. Zu den Pilzen gehören insbesondere Hefe, Schimmelpilze, Haut-, Haar- und Sproßpilze. Der Begriff "Bakterien" bedeutet Mikroorganismen mit heterotrophen oder autotrophen Stoffwechsel, die keinen Chromosomenkern aufweisen. Zu den Bakterien gehören Gram positive und Gram negative Mikroorganismen, insbesondere solche die auf der Hautoberfläche von Menschen oder Tieren wachsen können, beispielsweise hautpathogen Bakterien aus den Gattungen Staphylokokken, Streptokokken, Corynebakterien, Propionibacterien und Proteus sowie andere aerob und anaerob wachsende Bakterien wie Enterokokken, Escherichia coli, Pseudomonas und Klebsiellen. Der Begriff "Antibiotikaresistenz" bedeutet die Eigenschaft von Mikrorganismen gegen die therapeutisch erreichbare Wirkstoffkonzentration eines Wirkstoffs unempfindlich zu sein.

Die obengenannten Verbindungen der Formel I können sowohl in freier Form als auch als Salze eingesetzt werden, die Verwendung in freier Form ist bevorzugt.

Kommen organische Basen zur Anwendung, so werden vorzugsweise schwere flüchtige Basen eingesetzt, beispielsweise niedrigmolekulare Alkanolamine wie Ethanolamin, Diethanolamin, N-Ethylethanolamin, N-Methyl-diethanolamin, Triäthanolamin, Diethylamino-ethanol, 2-Amino-2-methyl-n-propanol, Dimethylaminopropanol, 2-Amino-2-methyl-propandiol, Tri-isopropanolamin. Als weitere schwerer flüchtige Basen seien beispielsweise erwähnt Ethylendiamin, Hexametylendiamin, Morpholin, Piperidin, Piperazin, Cyclohexylamin, Tributylamin, Dodecylamin, N,N-Dimethyl-dodecylamin, Stearylamin, Oleylamin, Benzylamin, Dibenzylamin, N-Ethylbenzylamin, Dimethylstearylamin, N-Methylmorphotin, N-Methylpiperazin, 4-Methylcyclohexylamin, N-Hydroxyethyl-morpholin. Auch die Salze quartärer Ammoniumhydroxide wie Trimethylbenzyl-ammonium-hydroxid, Tetramethylammoniumhydroxid oder Tetraethylammoniumhydroxid können verwendet werden, ferner Guanidin und seine Abkömmlinge, insbesondere seine Alkylierungsprodukte. Es ist jedoch auch möglich, als Salzbildner beispielsweise niedrigmolekulare Alkylamine wie Methylamin, Ethylamin oder Triethylamin einzusetzen. Auch Salze mit anorganischen Kationen, beispielsweise Aikatisalze, insbesondere Natrium-, Kalium- oder Ammonium-Salze, Erdalkalisalze wie insbesondere das Magnesium- oder Calciumsalz, sowie Salze mit zwei- bis vierwertigen Kationen, beispielsweise das Zink-, Aluminium- oder Zirkon-Salz kommen für die erfindungsgemäß einzusetzenden Verbindungen in Betracht.

Die in den Zubereitungen einzusetzenden Wirkstoffe der Verbindung der Formel I können beispielsweise nach Verfahren gemäß US 2 540 218 hergestellt werden.

Für den erfindungsgemäßen Einsatz der genannten Verbindungen kommen flüssige bis halbfeste pharmazeutische Zubereitungen in Betracht, insbesondere Lösungen, Creme-, Salben- und Gelzubereitungen, wo letztere wegen ihrer erhöhten Wirkstofffreisetzung bevorzugt Verwendung finden. Die Herstellung dieser Zubereitungen erfolgt in an sich bekannter Weise unter Zugabe des erfindungsgemäß eingesetzten Wirkstoffs. Die erfindungsgemäßen Zubereitungen können von den oben genannten 1-Hydroxy-2-pyridonen eine Verbindung oder auch mehrere in Kombination enthalten.

In den erfindungsgemäßen Zubereitungen wird der Wirkstoff in Mengen eingearbeitet, die üblicherweise zwischen etwa 0,1 und etwa 5 %, vorzugsweise zwischen 0,5 und 1 %, liegen.

Mit den erfindungsgemäßen Arzneimitteln läßt sich bei der topischen Behandlung von Infektionen der Haut eine durchgreifende Heilung erzielen. Die erfindungsgemäßen Mittel können auch zur Behandlung der Akne, der Rosacea - eine Erkrankung noch ungeklärter Ätiologie - und von Erythrasma, einer durch Corynebacterium minutissimum hervorgerufene Pseudomykose der Haut, eingesetzt werden.

### Beispiel 1

Eine erfindungsgemäße Zubereitung weist folgende Zusammensetzung auf:

| | |
|---|---|
| 1-Hydroxy-4-methyl-6-(2,4,4-trimethylpentyl)-2(1H)pyridon | 0,50 % |
| Hydroxyethylcellulose | 1,50 % |
| Polyethylenglykol-7 Glycerylcocoat | 5,00 % |
| 1,2-Propylenglykol | 10,00 % |
| Isopropylalkohol | 20,00 % |
| Demineralisiertes Wasser | 63,00 % |

### Beispiel 2

Eine erfindungsgemäße Zubereitung weist folgende Zusammensetzung auf:

| | |
|---|---|
| 1-Hydroxy-4-methyl-6-cyclohexyl-2(1 H)pyridon Polyacrylsäurepolymer | 1,00 % |
| (z.B. Carbomer 934 P) | 0,70 % |
| Natriumhydroxid | 0,20 % |
| Natriumdioctylsulfosuccinat | 0,05 % |
| 2-Octyldodecanol | 7,50 % |
| Isopropylalkohol | 25,00 % |
| Demineralisiertes Wasser | 65,55 % |

### Beispiel 3

Eine erfindungsgemäße Zubereitung weist folgende Zusammensetzung auf:

| | |
|---|---|
| 1-Hydroxy-4-methyl-6-cyclohexyl-2(1H)pyridon Polyacrylsäurepolymer | 0,50 % |
| (z.B. Carbomer 940) | 0,50 % |
| Natriumhydroxid | 0,20 % |
| Polyoxyethylen(20)sorbitanmonostearat | 3,50 % |
| Isopropylmyristat | 10,00 % |
| Ethanol | 20,00 % |
| Demineralisiertes Wasser | 65,30 % |

### Beispiel 4

Eine erfindungsgemäße Zubereitung weist folgende Zusammensetzung auf:

| | |
|---|---|
| 1 -Hydroxy-4-methyl-6-(2,4,4-trimethylpentyl)-2(1H)-pyridon | 1,00 % |
| Hydroxypropylcellulose | 1,00% |
| 1,2-Propylenglykol | 2,50 % |
| Ethanol | 20,00 % |
| Demineralisiertes Wasser | 75,50 % |

### Beispiel 5

Eine erfindungsgemäße Zubereitung weist folgende Zusammensetzung auf:

| | |
|---|---|
| 1-Hydroxy-4-methyl-6-cyclohexyl-2(1H)pyridon | 1,00% |
| Isopropylalkohol | 25,00 % |
| Polyethylenglykol 400 | 5,00 % |
| Demineralisiertes Wasser | 69,00 % |

### Beispiel 6

Eine erfindungsgemäße Zubereitung weist folgende Zusammensetzung auf:

| | |
|---|---|
| 1-Hydroxy-4-methyl-6-(trimethyl-pentyl)-2(1H)pyridon | 1,00% |
| 2-Octyldocenol | 5,00 % |
| Paraffinöl | 5,00 % |
| Cetylalkohol | 5,00 % |
| Stearylalkohol | 5,00 % |
| Myristylalkohol | 5,00 % |
| Polyoxyethylen-20-sorbitanmonostearat | 3,00 % |
| Sorbitan monostearat | 2,00 % |
| Demineralisiertes Wasser | 69,00 % |

### Beispiel 7

Wirksamkeitsprüfung

Bestimmung der antibakteriellen Wirksamkeit von 1-Hydroxy-4-methyl-6-cyclohexyl-2(1H)pyridon gegenüber hautpathogenen Gram-positiven und Gram-negativen aeroben Bakterien.

Die Bestimmung der minimalen Hemmkonzentration (MHK) erfolgte im Agarverdünnungstest in Mueller-Hinton Agar. Der Wirkstoff wurde zunächst 10 %ig in Dimethylsulfoxid gelöst und anschließend in gleichen Stufen mit Agar jeweils auf das zweifache verdünnt, so daß im Endeffekt Konzentrationen zwischen 128 µg/ml und 1 µg/ml erhalten wurden. Übemachtkulturen der zu prüfenden Bakterienstämme wurden mit Flüssigmedium verdünnt und als Inokulum eingesetzt. Die Bakteriensuspensionen (1 x 10⁵ cfu/ml) wurden auf die Oberfläche der wirkstoffhaltigen Agarplatten aufgetragen. Mit Ausnahme der Methicillin-resistenten Stämme von Staphylococcus aureus (MRSA) und Staphylococcus epidermidis (MRSE) wurden die MHK Werte nach 24 Stunden bei 37 °C abgelesen (MRSA und MRSE: 48 Stunden bei 30 °C).

Die niedrigste Konzentration, bei der kein Wachstum zu beobachten war, wurde als MHK bezeichnet.

Die untersuchten antibiotikaresistenten Bakterien können mit bekanten Methoden aus Patienten oder aus Krankenhäusern isoliert werden, bei denen Antibiotikaresistenz festgestellt wurde. Die anderen genannten Bakterienarten können aufgrund ihres Art- und Gattungsnamens leicht von einem Fachmann isoliert werden oder aus einer Stammsammlung angefordert werden.

### Ergebnisse

In vitro Wirksamkeit von 1-Hydroxy-4-methyl-6-cyclohexyl-2(1H)pyridon gegenüber aeroben Bakterien

| **Gram-positive Stämme** | n = | MHK (µg/ml) (n =) |
|---|---|---|
| Staphylococcus aureus | 20 | 64 |
| S. aureus, methicillin-resistente, MRSA | 19 | 64 |
| S. aureus, ofloxacin-resistent, OFX^{r} | 16 | 64₍₈₎, 128₍₈₎ |
| | | |
| Staphylococcus epidermidis | 20 | 128 |
| S. epidermidis, methicillin-resistent, MRSE | 2 | 64 |
| S. epidermidis, ofloxacin-resistent, OFX^{r} | 4 | 64 |
| | | |
| Streptococcus pyogenes | 20 | 64 |
| Strept. faecalis | 3 | 64₍₁₎, 128₍₂₎ |
| Strept. faecium | 1 | 128 |
| Strept. faecium, vancomycin-resistent, VAN^{r} | 1 | 32 |
| Strept. durans | 10 | 64₍₄₎, 128₍₆₎ |
| Strept. equisimilis | 1 | 128 |
| Strept. agalactiae | 9 | 128 |
| **Gram-negative Stämme** | | |
| Proteus vulgaris | 3 | 32₍₁₎, 64₍₂₎ |

| Gram-positive Stämme | n = | MHK (µg/ml) (n =) |
|---|---|---|
| Enterobacter aerogenes | 1 | 128 |
| Enterobacter cloacae | 1 | 128 |
| Escherichia coli | 3 | 64 |
| Klebsiella pneumoniae | 2 | 64₍₁₎, 128₍₁₎ |
| Pseudomonas aeruginosa | 5 | 128 |
| n = Anzahl der untersuchten Stämme; die in der Klammer genannte Zahl gibt die getesteten Stämme an bei der die genannte MHK ermittelt wurde. | | |

In vitro Wirksamkeit von 1-Hydroxy-4-methyl-6-cyclohexyl-2(1H)pyridon gegenüber anaeroben Bakterien (die Prüfung wurde im Agarverdünnungstest mit Wilkins-Chalgren-Agar (Oxoid) durchgeführt).

| **Bakterienbezeichnung** | | **MHK (µg/ml)** |
|---|---|---|
| Propionibacterium acnes | Stamm 6919 | 32,0 |
| " | Stamm 6922 | 32,0 |
| " | Stamm 15549 | 32,0 |
| " | Stamm DSM 20458 | 32,0 |

Alle geprüften Bakterienstämme werden- ohne Ausnahme - in einem sehr engen Konzentrationsbereich von 1-Hydroxy-2-pyridonen im Wachstum gehemmt. Dies gilt auch für Stämme, die gegen Antibiotika wie Methicillin, Ofloxacin und Vancomycin therapieresistent sind.

## Patentansprüche

1. Die Verwendung von 1-Hydroxy-2-pyridon der Formel I 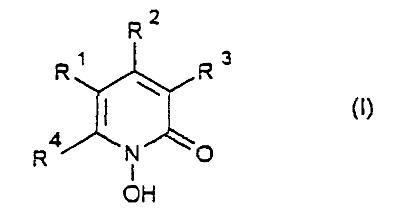 worin R¹, R² und R³, die gleich oder verschieden sind, Wasserstoffatom oder Alkyl mit 1 - 4 Kohlenstoffatomen bedeuten, und
R⁴ einen gesättigten Kohlenwasserstoffrest mit 6 bis 9 Kohlenstoffatomen oder einen Rest der Formel II bedeutet 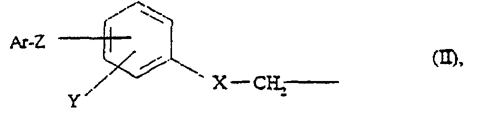 wobei
X S oder O bedeutet,
Y Wasserstoffatom oder bis zu 2 Halogenatome wie Chlor und/oder Brom bedeutet,
Z eine Einfachbindung oder die zweiwertigen Reste O, S, -CR²-(R = H oder (C₁-C₄)-Alkyl) oder andere zweiwertige Reste mit 2 - 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder (C₁-C₄)-Alkylgruppen abgesättigt sind, bedeutet,
Ar ein aromatisches Ringsystem mit bis zu zwei Ringen, das durch bis zu drei Reste aus der Gruppe Fluor, Chlor, Brom, Methoxy, (C₁-C₄)-Alkyl, Trifluormethyl und Trifluormethoxy substituiert sein kann, bedeutet,
zur Herstellung eines Arzneimittels zur Behandlung von Hautinfektionen, die durch Antibiotika resistente Bakterien verursacht werden.

2. Verwendung gemäß Anspruch 1, **dadurch gekennzeichnet, daß** man die Verbindung der Formel I einsetzt, worin Ar ein bicyclisches System darsteitt, das sich vom Biphenyl, Diphenylalkan oder Diphenylether ableitet.

3. Verwendung gemäß Anspruch 1 oder 2, **dadurch gekennzeichnet, daß** die Verbindung der Formel I in der Position R⁴ einen Cyclohexylrest enthält.

4. Verwendung gemäß einem oder mehreren der Ansprüche 1 bis 3, **dadurch gekennzeichnet, daß** die Verbindung der Formel I in der Position R⁴ einen Octylrest der Formel -CH₂-CH(CH₃)-CH₂-C(CH₃)₃ enthält.

5. Verwendung gemäß Anspruch 1, **dadurch gekennzeichnet, daß** man 1-Hydroxy-4-methyl-6-[4-(4-chlorphenoxy)-phenoxymethyl]-2-(1H)pyridon, 1-Hydroxy-4-methyl-6-cyclohexyl-2-(1H)pyridon oder 1-Hydroxy-4-methyl-6-(2,4,4-trimethylpentyl)-2-(1H)pyridon einsetzt.

6. Verwendung gemäß einem oder mehreren der Ansprüche 1 bis 5, **dadurch gekennzeichnet, daß** die Behandlung topisch erfolgt.

7. Verwendung gemäß Anspruch 6, **dadurch gekennzeichnet, daß** die Antibiotika resistenten Bakterien aus der Gruppe der Gram positive und/oder Gram negative Mikroorganismen stammen, insbesondere solche die auf der Hautoberfläche von Menschen oder Tieren wachsen können.

8. Verwendung gemäß Anspruch 7 zur topischen Behandlung von Hautinfektionen, die durch hautpathogene Bakterien aus einer oder meherer der Gattungen Staphylokokken, Streptokokken, Proteus, Corynebakterien und Propionibakterien verursacht werden oder die durch andere aerob oder anaerob wachsende Bakterien wie Escherichia coli, Enterokokken, Pseudomonas oder Klebsiellen verursacht werden.

9. Verwendung gemäß einem oder mehreren der Ansprüche 1 bis 8, **dadurch gekennzeichnet, daß** Akne, Rosacea oder Erythrasma behandelt wird.

## Claims

1. The use of 1-hydroxy-2-pyridones of the formula I 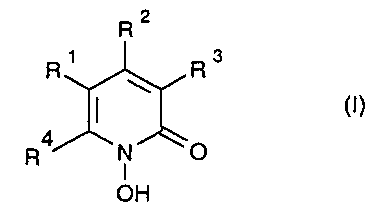 in which R¹, R² and R³, which are identical or different, are a hydrogen atom or alkyl having 1-4 carbon atoms, and
R⁴ is a saturated hydrocarbon radical having 6 to 9 carbon atoms or a radical of the formula II 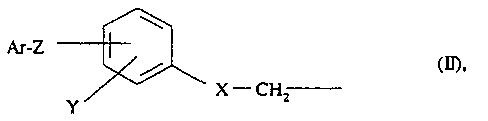 where
X is S or O,
Y is a hydrogen atom or up to 2 halogen atoms such as chlorine and/or bromine,
Z is a single bond or the divalent radicals O, S, -CR²- (R = H or (C₁-C₄)-alkyl) or other divalent radicals having 2-10 carbon and optionally O and/or S atoms linked in the form of a chain, where-if the radicals contain 2 or more O and/or S atoms - the latter must be separated from one another by at least 2 carbon atoms and where 2 adjacent carbon atoms can also be linked to one another by a double bond and the free valencies of the carbon atoms are saturated by H and/or (C₁-C₄)-alkyl groups,
Ar is an aromatic ring system having up to two rings which can be substituted by up to three radicals from the group consisting of fluorine, chlorine, bromine, methoxy, (C₁-C₄)-alkyl, trifluoromethyl and trifluoromethoxy,
for the production of a pharmaceutical for the treatment of skin infections which are caused by antibiotic-resistant bacteria.

2. The use as claimed in claim 1, wherein the compound of the formula I is employed in which Ar is a bicyclic system which is derived from biphenyl, diphenylalkane or diphenyl ether.

3. The use as claimed in claim 1 or 2, wherein the compound of the formula I contains a cyclohexyl radical in the position R⁴.

4. The use as claimed in one or more of claims 1 to 3, wherein the compound of the formula I contains an octyl radical of the formula -CH₂-CH(CH₃)-CH₂-C(CH₃)₃ in the position R⁴.

5. The use as claimed in claim 1, wherein 1-hydroxy-4-methyl-6-[4-(4-chlorophenoxy)phenoxymethyl]-2-(1H)pyridone, 1-hydroxy-4-methyl-6-cyclohexyl-2-(1H)pyridone or 1-hydroxy-4-methyl-6-(2,4,4-trimethylpentyl)-2-(1 H)pyridone is employed.

6. The use as claimed in one or more of claims 1 to 5, wherein treatment takes place topically.

7. The use as claimed in claim 6, wherein the antibiotic-resistant bacteria originate from the group comprising gram-positive and/or gram-negative microorganisms, in particular those which can grow on the skin surface of humans or animals.

8. The use as claimed in claim 7 for the topical treatment of skin infections which are caused by skin-pathogenic bacteria from one or more of the genera staphylococci, streptococci, Proteus, corynebacteria and propionibacteria or which are caused by other bacteria which grow aerobically or anaerobically, such as Escherichia coli, enterococci, Pseudomonas or Klebsiella.

9. The use as claimed in one or more of claims 1 to 8, wherein acne, rosacea or erythrasma is treated.

## Revendications

1. Utilisation de la 1-hydroxy-2-pyridone de formule I 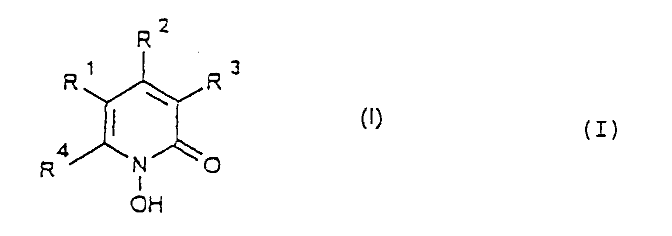 où
R¹, R² et R³, qui sont identiques ou différents, représentent un atome d'hydrogène ou un groupe alkyle présentant 1 à 4 atomes de carbone, et
R⁴ représente un reste hydrocarboné saturé présentant 6 à 9 atomes de carbone ou un reste de formule II 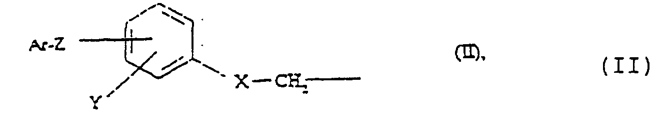
où
X représente un atome de S ou de O,
Y représente un atome d'hydrogène ou jusqu'à 2 atomes d'halogène, comme le chlore et/ou le brome,
Z représente une liaison simple ou les restes bivalents O, S, -CR²- (R = H ou un groupe alkyle en C₁-C₄) ou d'autres restes bivalents présentant 2 à 10 atomes de carbone reliés en chaîne et éventuellement des atomes de O et/ou S, où-lorsque les restes contiennent 2 ou plus d'atomes de O et/ou S - ces derniers doivent être séparés l'un de l'autre par au moins 2 atomes de carbone et où 2 atomes de carbone adjacents peuvent être également reliés les uns aux autres par une double liaison et les valences libres des atomes de carbone sont saturés par H et/ou groupes alkyle en C₁-C₄,
Ar représente un système cyclique aromatique présentant jusqu'à 2 cycles, qui peut être substitué par jusqu'à 3 restes pris dans le groupe comportant des atomes de fluor, de chlore, de brome, des restes méthoxy, alkyle en C₁-C₄, trifluorométhyle et trifluorométhoxy, sous forme libre ou salifiée,
pour la préparation d'un médicament pour le traitement local des infections de la peau qui sont provoquées par des bactéries résistantes aux antibiotiques.

2. Utilisation selon la revendication 1, **caractérisée en ce qu'**on utilise le composé de formule I, où Ar représente un système bicylique dérivant du biphényle, d'un diphénylalcane ou de l'éther diphénylique.

3. Utilisation selon la revendication 1 ou 2, **caractérisée en ce que** le composé de formule I présente en position R⁴ un reste cyclohexyle.

4. Utilisation selon une ou plusieurs des revendications 1 à 3, **caractérisé en ce que** le composé de formule I présente en position R⁴ un reste octyle de formule -CH₂-CH(CH₃)-CH₂-C(CH₃)₃.

5. Utilisation selon la revendication 1, **caractérisée en ce qu'**on utilise la 1-hydroxy-4-méthyl-6-[4-(4-chlorophénoxy)-phénoxyméthyl]-2-(1H)-pyridone, la 1-hydroxy-4-méthyl-6-cyclohexyl-2-(1H)-pyridone ou la 1-hydroxy-4-méthyl-6-(2,4,4-triméthylpentyl)-2-(1H)-pyridone.

6. Utilisation selon une ou plusieurs des revendications 1 à 5, **caractérisée en ce qu'**on effectue le traitement par voie locale.

7. Utilisation selon la revendication 6, **caractérisée en ce que** les bactéries résistantes aux antibiotiques appartiennent au groupe de micro-organismes gram-positifs et/ou gram-négatifs, en particulier ceux qui peuvent pousser sur la surface de la peau de l'homme ou des animaux.

8. Utilisation selon la revendication 7 pour le traitement local d'infections de la peau qui sont provoquées par des bactéries pathogènes pour la peau d'une ou plusieurs espèces de *Staphylocoques,* de *Streptocoques,* de *Proteus,* de *Corynebactéries* et de *Propionibactéries* ou qui sont provoquées par d'autres bactéries qui poussent en aérobies ou en aérobie comme *Escherichia coli, Entérocoques, Pseudomonas* ou *Klebsiella.*

9. Utilisation selon une ou plusieurs des revendications 1 à 8, **caractérisée en ce qu'**on traite l'acné, la rosacée ou l'érythrasma.
